# EUROPÄISCHE PATENTANMELDUNG

(11) **EP 0 620 435 A2**
(43) Veröffentlichungstag der Anmeldung: **19.10.1994**
(21) Anmeldenummer: 94250071.1
(22) Anmeldetag: 22.03.1994
(51) Int. Cl.: G01N 33/00, B01D 53/26

(54) **Einrichtung zur Aufbereitung eines Analysegases**

(30) Priorität: 13.04.1993 DE 4312907
(71) Anmelder: MANNESMANN Aktiengesellschaft, D-40213 Düsseldorf (DE)
(72) Erfinder: Helfrich, Helmut, D-63679 Schotten-Betzenrod (DE); Vogt, Siegfried, D-69190 Walldorf (DE); Winter, Lothar, D-63633 Birstein (DE)
(74) Vertreter: Presting, Hans-Joachim, Dipl.-Ing.

(57) **Zusammenfassung**

Beschrieben wird eine Einrichtung (11) zur Aufbereitung eines Analysegases, mit einem nach dem Prinzip der Permeationsdestillation arbeitenden Kondensatabscheider welcher im wesentlichen aus einem Außenrohr (22) mit innenliegendem und aus Wasserdampf-permeablem Material bestehenden Innenrohr (23) besteht. Um bei einer solchen Einrichtung den Wirkungsgrad der Kondensatabscheidung optimal konstant zu halten, ist vorgeschlagen, daß der Kondensatabscheider nach außen thermisch isoliert in einem Behältnis (20) angeordnet und mit einem thermostatisierten Kühler (26) thermisch gekoppelt ist.

## Beschreibung

Die Erfindung betrifft eine Einrichtung zur Aufbereitung eines Analysegases, mit einem nach dem Prinzip der Permeationsdestillation arbeitenden Kondensatabscheiders gemäß Oberbegriff des Patentanspruches 1.

Eine Einrichtung dieser Art ist aus der DE 37 16 350 C2 bekannt. Hierbei besteht der Kondensatabscheider aus einem Außenrohr, in welchem ein aus Wasserdampf-permeablem Material bestehendes Innenrohr angeordnet ist. Dabei wird das Analysegas bzw. das zu analysierende Gas durch das Innenrohr geleitet, wobei durch das Außenrohr ein Spülgas, zumeist im Gegenstrom, geführt wird. Die Ausbildung des Innenrohres aus Wasserdampf-permeablem Material läßt nun eine Permeation des innerhalb des Analysegases enthaltenen Wasserdampf es zu, so daß auf diese Weise das Analysegas vom Wasserdampf befreit werden kann.

Der Wasserdampf wird dabei über ein im Außenrohr eingebrachtes Spülgas aus dem Kondensatabscheider abgesaugt. Solche Einrichtungen werden deshalb zur Aufbereitung von Analysegasen verwendet, da die meisten optischen Analysemeßeinrichtungen bezüglich einiger Meßgase wasserdampfquerempfindlich sind. Wasserdampfquerempfindlichkeit bedeutet, daß der im Analysegas enthaltene Wasserdampf im Detektor der Analyseeinrichtung den gleichen Meßeffekt erzeugt wie die spezifische Meßgaskomponente, auf die der Detektor empfindlich ist.

Der Wirkungsgrad der Kondensatabscheidung bei der Permeationsdestillation ist aus physikalischen und chemischen Gründen temperaturabhängig. Dies ergibt sich aus der Temperaturabhängigkeit der hygroskopischen Eigenschaft der verwendeten wasserdampfpermeablen Materialien. Beim Einsatz solcher Kondensatabscheider zur Aufbereitung von Analysegasen kann eine solche Temperaturabhängigkeit beispielsweise bei wechselnden Umgebungstemperaturen durch den Abscheider zu Schwankungen des Wirkungsgrades führen, die das Meßergebnis der Analyse verfälschen können.

Der Erfindung liegt daher die Aufgabe zugrunde eine Einrichtung zur Aufbereitung eines Analysegases nach dem Prinzip der Permeationsdestillation derart weiterzubilden, daß der Wirkungsgrad der Kondensatabscheidung möglichst konstant bleibt.

Die gestellte Aufgabe wird bei einer Einrichtung der gattungsgemäßen Art erfindungsgemäß dadurch gelöst, daß der Kondensatabscheider nach außen thermisch isoliert in einem Behältnis angeordnet ist und innerhalb des Behältnisses mit einem thermostatisierten Kühler thermisch gekoppelt ist.

Hierdurch kann nun erreicht werden, daß unabhängig von den Umgebungstemperaturen des Analysegas, der Kondensatabscheider auf einem konstant gleichen Wirkungsgrad gehalten werden kann. Dabei kann als Kühler entweder ein Peltierkühler oder ein Kompressorkühler eingesetzt werden. In besonderer Ausgestaltung der Erfindung ist das Außenrohr des Kondensatabscheiders mitsamt innenliegendem Rohres um einen Zylinder derart gewickelt, daß das Außenrohr in einer gewindeähnlich verlaufenden Nut im Mantelbereich des Zylinders eingelegt ist. Die gesamte Anordnung, daß heißt Kondensatabscheider mit Zylinder ist dann innerhalb eines thermisch isolierenden Behältnisses angeordnet, wobei mindestens an einer Stirnseite des Zylinders ein thermisch gekoppelter Pertierkühler angeordnet ist. Bei der Verwendung eines Kompressorkühlers ist vorgesehen, daß der Kondensatabscheider wieder mitsamt dem Zylinder von einer dünnen Zylinderhülle umgeben ist, wobei um die Zylinderhülle die Kühlschlangen des Kompressorkühlers angeordnet sind. Die gesamte Anordnung, daß heißt Kompressorkühler und Kondensatabscheider mit Zylinder sind innerhalb des Behältnisses thermisch isoliert angeordnet.

Vorzugsweise wird der Zylinder, und damit der Kondensatabscheider auf eine Temperatur von 2 bis 10 Grad thermostatisiert gehalten. In der Regel sind solchen Permeationsdestillationstrocknern noch Kühlstufen vorgeschaltet. Durch die oben genannte thermostatisierte Temperatur des Kondensatabscheiders können somit konstant tiefe Taupunkte von kleiner oder gleich minus 30 Grad gehalten werden. Dabei ist außerdem zu beachten, daß im Innenrohr, welches das Analysegas enthält, von welchem der Wasserdampf getrennt werden soll, etwa ein Druckniveau von absolut 3 bar, und im Außenrohr ein Absolutdruck von etwa 300 millibar gehalten wird. Übliche Durchflußmengen sind dabei etwa U=60 Liter pro Stunde, das Druckverhältnis beträgt etwa 9:1, und bei der oben eingestellten thermostatisierten Temperatur des Zylinders entstehen dann die genannten Taupunkte im Bereich von kleiner oder gleich minus 30 Grad. Mit diesen Parametern ist es möglich auf einen Wasserdampfanteil von weniger als 375 ppm H20 zu gelangen. Der Taupunkt kann konstant gehalten werden und der Kondensatabscheider gewährleistet somit stets die optimale Wasserdampftrennung. Der Einfluß der Querempfindlichkeit auf die zur Analyse nachgeschaltete Analyseeinrichtung ist somit konstant minimiert. Die Erfindung ist in der Zeichnung im nachfolgenen näher beschrieben. Es zeigt:
Figur 1 Kondensatabscheider mit Peltierkühler
Figur 2 Kondensatabscheider mit Kompressorkühler
Figur 3 Gesamtanordnung der Analysegasaufbereitung.

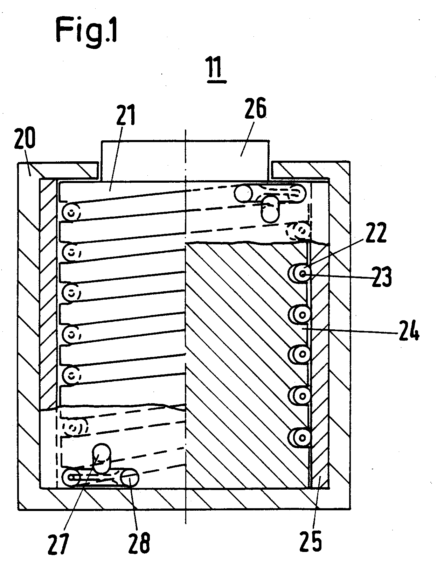

Figur 1 zeigt eine erste Ausgestaltungsversion der Erfindung, wobei als Kühler ein Peltierkühler 26 eingesetzt ist. Der Kondensatabscheider ist in einem thermisch isolierenden Behältnis 20 angeordnet. Das Außen- und Innenrohr 22, 23 des eigentlichen Kondensatabscheiders ist um einen Zylinder 21 gewickelt, innerhalb des Behältnisses 20 angeordnet. Um das Außenrohr 22 dabei ortsfest zu halten ist der Zylinder im Außenmantelbereich mit einer gewindeförmig verlaufenden Nut 24 versehen, in die das Außenrohr eingelegt ist. Die Nut 24 ist dabei vorzugsweise so tief zu wählen, daß das Außenrohr 22 ganz aufgenommen werden kann. Um den mit dem Kondensatabscheider versehenen Zylinder 21 ist eine Zylinderhülle 25 angeordnet, wobei die gesamte Anordnung bestehend aus Kondensatabscheider, Zylinder 21 und Hülle 25 innerhalb des Behältnisses 20 angeordnet ist. An den Enden des Außenrohres 22 und des Innenrohres 23 ist jeweils ein Spülgas Anschluß 27 und ein Analysegas Anschluß 28. Das Analysegas wird über den Analysegasanschluß 28 dem Innenrohr 23 des Kondensatabscheiders zugeführt. Das Innenrohr 23 besteht dabei aus einem Wasserdampf-permeablem Material. Das Außenrohr 22 wird dabei über den Spülgasanschluß 27 mit Spülgas, welches zumeist in Gegenstrom gegen die Analysegasführung geführt wird, gespeist. Dieses Prinzip ist das an sich bekannte Prinzip der Permeationsdestillation und braucht hier nicht weiter beschrieben zu werden.

An einem stirnseitigen Ende des Zylinders ist ein Peltierkühler 26 angeordnet, der mit dem Zylinder 21 thermisch gekoppelt ist, um somit den Kondensatabscheider thermostatisiert auf einer je nach Betriebsparameter wählbaren Temperatur zu halten.

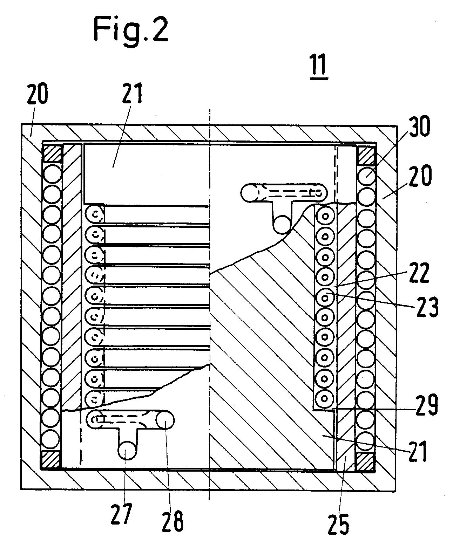

Figur 2 zeigt eine alternative Ausgestaltungsmöglichkeit der Erfindung, bei der ein Kompressorkühler eingesetzt ist. Das Außenrohr 22 mitsamt dem Innenrohr 23 ist hierbei ebenfalls um einen Zylinder 21 herum angeordnet, "gewickelt". Hierbei ist jedoch keine gewindeförmige umlaufende Nut wie in Figur 1 vorgesehen, sondern der Zylinder ist über einen Abschnitt mit einer breiten ringförmigen Ausnehmung 29 versehen, in welcher das Außenrohr 22 mitsamt dem Innenrohr 23 des Kondensatabscheiders eingelegt ist. Natürlich sind wieder jeweils ein Spülgasanschluß 27 und ein Analysegasanschluß 28 vorgesehen. Diese Anschlüsse sind, wie in Figur 1 ebenfalls dargestellt, an beiden Enden des Außen bzw. Innenrohres des Kondensatabscheiders angeordnet. Der Kondensatabscheider mitsamt Zylinder 21 ist auch hierbei von einer Zylinderhülle 25 umgeben, um die außen herum die Kühlschlagen eines Kompressorkühlers 30 angelegt sind. Kompressorkühler 30 sowie Zylinderhülle 25 und Zylinder 21 mit Außen- und Innenrohr 22, 23 sowie die Anschlüsse 27 und 28 sind auch hierbei innerhalb eines nach außen thermisch isolierten Behältnisses 20 angeordnet.

Bei beiden Ausgestaltungsmöglichkeiten ist das Wesen der Erfindung realisiert, nämlich den Kondensatabscheider nach außen thermisch zu isolieren, und innen thermostatisiert zu kühlen. Hierbei kann der Kondensatabscheider auf einer festen Temperatur gehalten werden, so daß ein konstant tiefer Taupunkt erreicht werden kann. Die gesamte Anordnung ist damit Wirkungsgrad-stabil, und eignet sich vorzüglich für den Einsatz nachgeschalteter empfindlicher Meßgasdetektoren. Der Querempfindlichkeitseinfluß bezüglich Wasserdampf ist auf ein Mindestmaß reduziert.

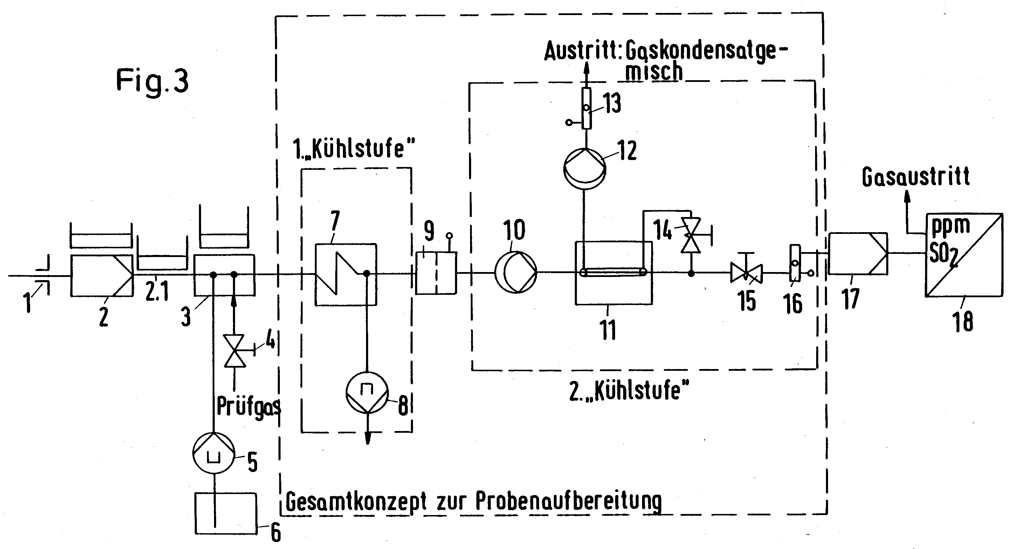

Figur 3 zeigt die gesamte Einbindung des Kondensatabscheiders in Gesamtübersicht aller eingesetzten Geräte bei der Verwendung in der Analysetechnik.

Über eine Entnahmesonde 1 wird zunächst Analysegas aufgenommen und einen Außenfilter 2 mit Heizung zugeführt. Über eine beheizbare Meßgasleitung 2.1 wird das Analysegas in einen sogenannten Gaswäscher 3, welcher beheizt ist, eingebracht. Der Gaswäscher 3 ist dabei noch mit einem Absperrventil 4 und einer Reagenzpumpe 5 mit nachgeordnetem Reagenzbehälter 6 mit Schwimmerschalter versehen. Vom Gaswäscher 3 gelangt nun das Analysegas in einen Meßgaskühler 7. Der Meßgaskühler 7 ist dabei mit einem automatischen Kondensatablaß 8 versehen. Weiterhin gelangt das Meßgas von dem Meßgaskühler 7 über einen Kondensatwächter 9 und eine Membranpumpe 10 in den Kondensatabscheider 11, welcher beispielsweise mit einem Peltierkühler versehen ist und vorzugsweise auf einer Temperatur von plus 2 Grad gehalten ist. Über eine Membranvakuumpumpen wird der Zwischenraum zwischen Außenrohr und Innenrohr des Kondensatabscheiders auf ein Druckniveau von etwa 300 millibar abgepumpt. Hierüber erfolgt dann der Austritt des Spülgas- und Kondensatgemisches über einen Durchflußwächter 13. Am Ausgang des Kondensatabscheiders sind Ventile 14 und 15 vorgesehen die vorzugsweise als Nadelventile ausgestaltet sind. Von da aus gelangt das Analysegas, welches nun dem Wasserdampf entzogen ist über einen Durchflußwächter 16 zu einem Einwegfilter 17 und von dort aus sofort zum Analysator 18. Die eigentliche Meßgasaufbereitung erfolgt in einer ersten Kühlstufe, welche von dem Meßgaskühler7 gebildet wird, und einer zweiten Kühlstufe die im wesentlichen den Kondensatabscheider 11 enthält.

## Patentansprüche

1. Einrichtung zur Aufbereitung eines Analysegases, mit einem nach dem Prinzip der Permeationsdestillation arbeitenden Kondensatabscheider, welcher im wesentlichen aus einem Außenrohr mit innenliegendem und aus Wasserdampf-permeablem Material bestehenden Innenrohr besteht,
dadurch gekennzeichnet,
daß der Kondensatabscheider nach außen thermisch isoliert in einem Behältnis (20) angeordnet und mit einem thermostatisierten Kühler (26, 30) thermisch gekoppelt ist.

2. Einrichtung zur Aufbereitung eines Analysegases nach Anspruch 1,
dadurch gekennzeichnet,
daß das Außenrohr (22) des Kondensatabscheiders in mehreren Windungen um einen Zylinder (21) gewickelt und in dem Behältnis (20) angeordnet ist, und der Zylinder (21) mit dem thermostatisierten Kühler (26, 30) gekoppelt ist.

3. Einrichtung zur Aufbereitung eines Analysegases nach Anspruch 2,
dadurch gekennzeichnet,
daß der Zylinder (21) im Außenmantel eine gewindeähnlich verlaufende Nut (24) enthält, in welche das Außenrohr (22) des Kondensatabscheiders eingelegt ist.

4. Einrichtung zur Aufbereitung eines Analysegases nach Anspruch 3,
dadurch gekennzeichnet,
daß an mindestens einer der Stirnseiten des Zylinders (21) der thermostatisierte Kühler (26) thermisch gekoppelt angeordnet ist.

5. Einrichtung zur Aufbereitung eines Analysegases nach Anspruch 4,
dadurch gekennzeichnet,
daß der Kühler aus einem Peltierkühler (26) besteht.

6. Einrichtung zur Aufbereitung eines Analysegases nach Anspruch 4,
dadurch gekennzeichnet,
daß der Zylinder (21) abschnittsweise mit einer ringförmigen Ausnehmung (29) versehen ist, in welche das Außenrohr (22) des Kondensatabscheiders eingelegt ist.

7. Einrichtung zur Aufbereitung eines Analysegases nach Anspruch 6,
dadurch gekennzeichnet,
daß der Zylinder (21) insgesamt von der Kühlschlange (30) eines Kompressorkühlers umgeben ist.

8. Einrichtung zur Aufbereitung eines Analysegases nach Anspruch 7,
dadurch gekennzeichnet,
daß zwischen dem vom Außenrohr (22) des Kondensatabscheiders umgebenen Zylinder (21) und der Kühlschlange (30) des Kompressorkühlers eine Zylinderhülle (25) angeordnet ist, und die gesamte Anordnung mit Kondensatabscheider und Kompressorkühlschlange (30) innerhalb des Behältnisses (20) angeordnet ist.
